# EUROPEAN PATENT APPLICATION

(11) **EP 4 497 398 A1**
(43) Date of publication of application: **29.01.2025**
(21) Application number: 22819144.1
(22) Date of filing: 21.03.2022
(51) Int. Cl.: A61B 17/122

(54) **PLUG-IN CLAMPING INSTRUMENT AND CLAMPING MEMBER THEREOF**

(71) Applicant: Ningbo Xinwell Medical Technology Co., Ltd., Cixi, Zhejiang 315334 (CN)
(72) Inventor: HUANG, Junjun, Cixi, Zhejiang 315334 (CN); SHAN, Jian, Cixi, Zhejiang 315334 (CN); WU, Hailiang, Cixi, Zhejiang 315334 (CN); CHEN, Qingye, Cixi, Zhejiang 315334 (CN); SUN, Zhongli, Cixi, Zhejiang 315334 (CN)
(74) Representative: Murgitroyd & Company
(86) International application number: PCT/CN2022/082112
(87) International publication number: WO 2022/257539

(57) **Abstract**

A plug-in clamping instrument and a clamping member thereof. The clamping member (100) comprises at least two clamping arms (110). Each clamping arm (110) comprises a clamping head (1111) and a bendable part (1112). The clamping arms (110) are used to clamp a target object. Each bendable part (1112) is provided with a deformation structure capable of bending towards the closing direction of the clamping member (100) and/or bending towards the opening direction of the clamping member (100). In the present structure, the sleeve in existing structures is omitted, and in combination with the deformation state of the bendable parts (1112), the clamping members (100) open and close on the basis of the position change of moving members (200). Given a same requirement for the opening range, the length of the clamping members (100) is shorter than the combination of the clamping arm (110) and the sleeve in the prior art. By using the clamping members (100), the overall clamping device has fewer parts, the structure is simpler, the assembly requirement is lower, and the costs are greatly reduced.

## Description

### TECHNICAL FIELD

The present application relates to the field of medical devices, and in particular to a structure of an insertable tissue clamp device for surgery.

### BACKGROUND

An insertable gripping instrument is an insertable medical device, including a clamp device configured to clamp tissue in a human body or an animal body (collectively referred to as a subject) and temporarily retain a gripping head in the subject after surgery, such as a hemostatic clamp or a tissue clamp, and further including a gripping device configured to grip the tissue in the body of the subject during surgery (where the gripping head is not required to be retained in the body of the subject after surgery), such as a clamp used in minimally invasive surgery.

An existing gripping instruction has a complex structure and a large size. For example, the clamp device is a hemostatic clamp (or a tissue clamp). One type of hemostatic clamps mainly achieves opening and gripping through coordination between gripping arms and a sleeve. Specifically, left and right gripping arms are loosely assembled together through a pin, and the gripping arms can retract into the sleeve to achieve closing. The gripping arms may also extend out of the sleeve to achieve opening. In such structure, since the closing of the gripping arms is realized by using an axial space of the sleeve, part of the gripping arms is required to be retracted in the sleeve, resulting in a longer overall length of the hemostatic clamp retained in a patient after separation, which is more likely to cause damage and discomfort to the patient.

In another type of hemostatic clamps (or tissue clamps), gripping arms thereof are connected mainly by a rotating shaft, and then the sleeve is provided with a rail for sliding up and down. The shaft may slide along the rail. A fixed shaft is disposed at the upper end of the sleeve. Elongated holes are disposed on the gripping arms. The fixed shaft extends through the elongated holes in the gripping arms. The two gripping arms are driven to move up and down by pushing and pulling the shaft to slide. After being blocked by the fixed shaft, the gripping arms are forced to move along paths in the elongated holes, thereby realizing opening and closing. The structure improves control accuracy, and a size of a clamp becomes smaller. However, there are more parts, the structure becomes complex, and the cost is higher.

### SUMMARY

The present application provides an insertable tissue clamp device and a gripper thereof, to demonstrate a new opening and gripping structure.

Based on the above objects, in an embodiment of the present application, an insertable gripping instrument is provided, including:
a gripper, the gripper including at least two gripping arms, the gripping arms being connected to each other, each gripping arm including a gripping head and a bendable portion, the gripping head and the bendable portion being connected to each other, and the gripping arm being configured to grip a target; the bendable portion having a deformable structure capable of bending in a closing direction of the gripper and/or bending in an opening direction of the gripper; and
a moving member movably arranged and being connected to the gripper, the gripper opening and closing based on a change in a position of the moving member.

In an embodiment, the deformable structure includes a plurality of first shrinkage slits. The first shrinkage slits are arranged sequentially in a longitudinal direction of the gripper.

In an embodiment, the first shrinkage slits are divided into a plurality of groups. Each group of first shrinkage slits has at least one first shrinkage slit. The bendable portion has a plurality of second shrinkage slits extending in a circumferential direction of the bendable portion. The second shrinkage slits are arranged in the longitudinal direction. Two ends of each group of first shrinkage slits respectively extend between two adjacent second shrinkage slits in the longitudinal direction. An overlapping region between the first shrinkage slits and the second shrinkage slits form a twist deformation section, to cause the bendable portion to be capable of bending and twist deformation.

In an embodiment, each of the limiting structures includes a plurality of limiting elements arranged in the longitudinal direction of the gripper. The limiting elements include first limiting blocks and second limiting blocks that face each other. Gaps arranged in the longitudinal direction are provided between the first limiting blocks and the second limiting blocks. The second shrinkage slits are in communication with the gaps. During a bending of the bendable portion in the opening direction, the first limiting blocks and the second limiting blocks approach each other and form a buckling structure.

In an embodiment, in a same limiting element, each first limiting block and each second limiting block are formed by cutting in sidewalls of the bendable portion located on sides of a respective one of the first shrinkage slits, through the respective second shrinkage slits. First ends of the first limiting block and the second limiting block adjacent to the first shrinkage slit are connected into an entirety, and second ends of the first limiting block and the second limiting block are separated from each other.

In an embodiment, an initial state of the gripper is a gripping state. The second shrinkage slit has a gap in the longitudinal direction. The second shrinkage slits form an adaptive floating structure capable of being deformed in a gripping direction, so that, when the gripper grips the target, the bendable portion is capable of being adaptively bent and deformed in the closing direction according to a volume of the target.

Based on the above object, a gripper for an insertable gripping instrument is provided in an embodiment of the present application. The gripper includes at least two gripping arms. The gripping arms are connected to each other. Each of the gripping arms includes a gripping head and a bendable portion. The gripping head and the bendable portion are connected to each other. The gripping arm is configured to grip a target. The bendable portion has a deformable structure capable of bending in a closing direction of the gripper and/or bending in an opening direction of the gripper.

In an embodiment, the deformable structure includes a plurality of first shrinkage slits. The first shrinkage slits are arranged sequentially in a longitudinal direction of the gripper.

In an embodiment, the bendable portion has a plurality of second shrinkage slits extending in a circumferential direction of the bendable portion. The second shrinkage slits are arranged in the longitudinal direction. Two ends of each group of first shrinkage slits respectively extend between two adjacent second shrinkage slits in the longitudinal direction. An overlapping region between the first shrinkage slits and the second shrinkage slits form a twist deformation section, to cause the bendable portion to be capable of bending and twist deformation.

In an embodiment, each limiting structure include a plurality of limiting elements arranged in the longitudinal direction of the gripper. The limiting elements include: first limiting blocks and second limiting blocks that face each other. Gaps arranged in the longitudinal direction are provided between the first limiting blocks and the second limiting blocks, and the second shrinkage slits are in communication with the gaps. During a bending of the bendable portion in the opening direction, the first limiting blocks and the second limiting blocks approach each other and form a buckling structure.

In an embodiment, in a same limiting element, each first limiting block and each second limiting block are formed by cutting in sidewalls of the bendable portion located on sides of a respective one of the first shrinkage slits, through the respective second shrinkage slits. First ends of the first limiting block and the second limiting block adjacent to the first shrinkage slit are connected into an entirety, and second ends of the first limiting block and the second limiting block are separated from each other.

In an embodiment, an initial state of the gripper is a gripping state; each second shrinkage slit has a gap in the longitudinal direction. The second shrinkage slits in the limiting structures form an adaptive floating structure capable of deforming in a gripping direction, so that, when the gripper grips the target, the bendable portion is capable of being adaptively bent and deformed in the closing direction according to a volume of the target.

According to the insertable tissue clamp device in the above embodiments, the gripper thereof includes at least two gripping arms, and each gripping arm includes the gripping head and the bendable portion. The gripping arms are arranged in a clamping jaw structure to grip a target. The bendable portion has the deformable structure capable of bending in a closing direction of the gripper and/or bending in an opening direction of the gripper. In such structure, the sleeve in the existing structure is omitted, and with the deformation state of the bendable portion, the gripper can open and close based on a change in the position of the moving member. Under the same open width requirement, the length of the gripper is shorter than that of a structure combing the gripping arms and the sleeve. After the gripper is adopted, the entire tissue clamp device has fewer parts, a simpler structure, lower assembly requirements, and a greatly reduced cost.

### BRIEF DESCRIPTION OF THE DRAWINGS

FIG. 1 is a schematic structural diagram of a gripper in a gripping state according to an embodiment of the present application.
FIG. 2 is a schematic structural diagram of the gripper shown in FIG. 1 in an opened state.
FIG. 3 is a schematic diagram showing assembling of a gripper having a split structure according to an embodiment of the present application, with the gripper in a gripping state.
FIG. 4 is a schematic structural diagram of the gripper shown in FIG. 3 in an opened state.
FIG. 5 is a schematic diagram showing assembling of a gripper having a split structure according to an embodiment of the present application, with the gripper in a gripping state.
FIG. 6 is a schematic structural diagram of the gripper moving to an opened state according to an embodiment of the present application.
FIG. 7 is a schematic structural diagram of the structure shown in FIG. 6, with a part thereof being cut away.
FIG. 8 is a schematic structural diagram of the gripper moving to the gripping state according to an embodiment of the present application.
FIG. 9 is a schematic structural diagram of the structure shown in FIG. 8, with a part thereof being cut away.
FIG. 10 is a schematic structural diagram of an integrally formed structure of a separation base and the gripper according to an embodiment of the present application, with the gripper in the gripping state.
FIG. 11 is a schematic structural diagram of the gripper shown in FIG. 10 in an opened state.
FIG. 12 is a schematic diagram of the gripper moving to the opened state according to an embodiment of the present application.
FIG. 13 is a schematic structural diagram of the structure shown in FIG. 12, with a part thereof being partially cut away.
FIG. 14 is a schematic structural diagram of the gripper moving to the gripping state according to an embodiment of the present application.
FIG. 15 is a schematic structural diagram of the structure shown in FIG. 14, with a part thereof being partially cut away.
FIG. 16 is a schematic diagram of an unfolding shape of the gripper according to an embodiment of the present application.
FIG. 17 is a schematic enlarged view of a deformable structure of a bendable portion in the embodiment shown in FIG. 16.
FIG. 18 is a schematic enlarged view of the deformable structure of the bendable portion according to another embodiment of the present application.
FIG. 19 is a schematic enlarged view of the deformable structure of the bendable portion according to another embodiment of the present application.
FIG. 20 is a schematic diagram of the bendable portion when thinner tissue is gripped according to an embodiment of the present application.
FIG. 21 is a schematic diagram of the bendable portion when thicker tissue is gripped according to an embodiment of the present application.
FIG. 22 is a schematic diagram of the deformable structure of the bendable portion according to another embodiment of the present application.
FIG. 23 is a schematic diagram of the deformable structure of the bendable portion according to another embodiment of the present application.
FIG. 24 is a schematic diagram of a gripping head in an opened and self-locked state according to an embodiment of the present application.
FIG. 25 is a schematic diagram of a moving member separated from the gripper according to an embodiment of the present application.
FIG. 26 is a schematic diagram of the gripper separated from a separation base according to an embodiment of the present application.
FIG. 27 is a schematic diagram of a retaining section separated from a separation section according to an embodiment of the present application.
FIG. 28 is a schematic diagram of the gripper separated from the separation base according to an embodiment of the present application.
FIG. 29 is a schematic structural diagram of an insertable gripping instrument according to an embodiment of the present application, with a transmission assembly depicted in a simplified manner.

### DETAILED DESCRIPTION

The present application is described in further detail below from specific embodiments with reference to the drawings.

This embodiment provides an insertable gripping instrument (for convenience of description, hereinafter referred to as a gripping instrument). The gripping instrument may be a clamp device such as a hemostatic clamp or a tissue clamp required to temporarily leave a gripping head in a human or animal body (collectively referred to as a subject) after surgery, or may be a gripping device such as a clamp (which not required to retain the gripping head in the body of the subject after surgery).

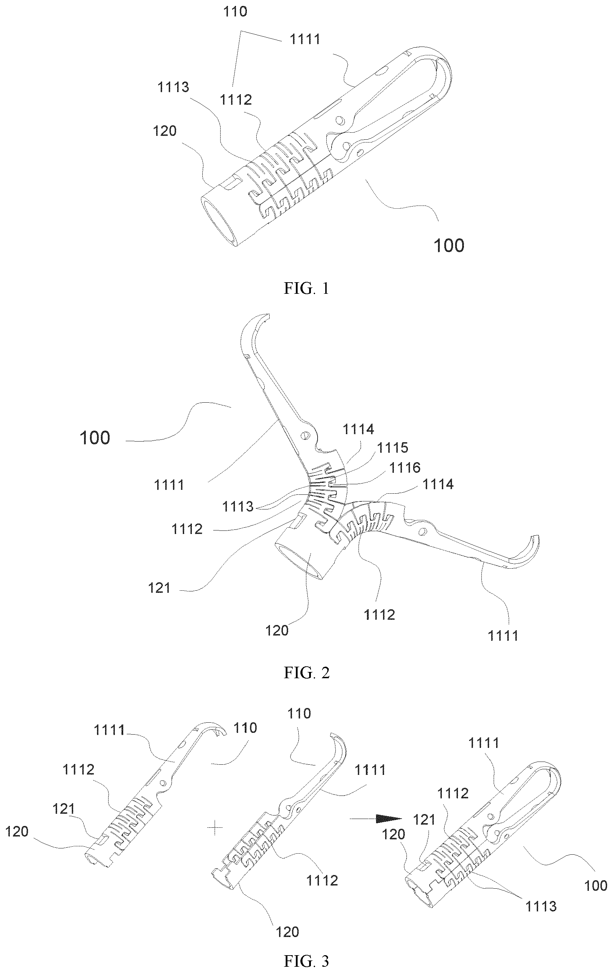

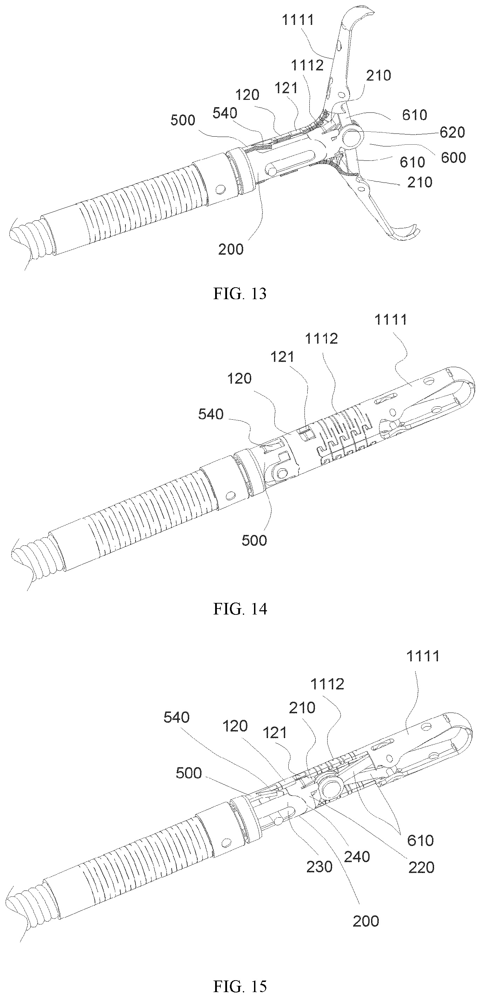

Referring to FIGS. 1 to 15, the gripping instrument includes a gripper 100, a moving member 200, and other related structures. The gripper 100 and the moving member 200 cooperate with each other to achieve gripping and opening actions.

Different from the prior art in which a gripping structure is realized by a combination of gripping arms and a sleeve, in this embodiment, the gripper 100 includes at least two gripping arms 110, The gripping arms 110 are connected. The gripping arms 110 may be integrally formed, or may be a fixedly connected into one piece. Each gripping arm 110 includes a gripping head 1111 and a bendable portion 1112. The gripping head 1111 is connected to the bendable portion 1112. Similarly, the gripping head 1111 and the bendable portion 1112 may be integrally formed or may be fixedly connected into one piece.

Specifically, referring to FIGS. 1 and 2, in an embodiment, the gripper 100 has an integrally formed structure. The gripping arms 110 have an integrally formed structure. In the same gripping arm 110, the gripping head 1111 and the bendable portion 1112 also have an integrally formed structure. The integrally formed structure means that the entire gripper 100 is integrally machined with the same material and is not assembled from two or more parts. The integrally formed structure (including other integrally formed structures described below) may be made by, but is not limited to, injection molding, laser cutting, or other machining processes. In particular, when laser cutting is used, extremely small gaps may be machined, which is beneficial to miniaturization of an overall structure and improvement of structural compactness.

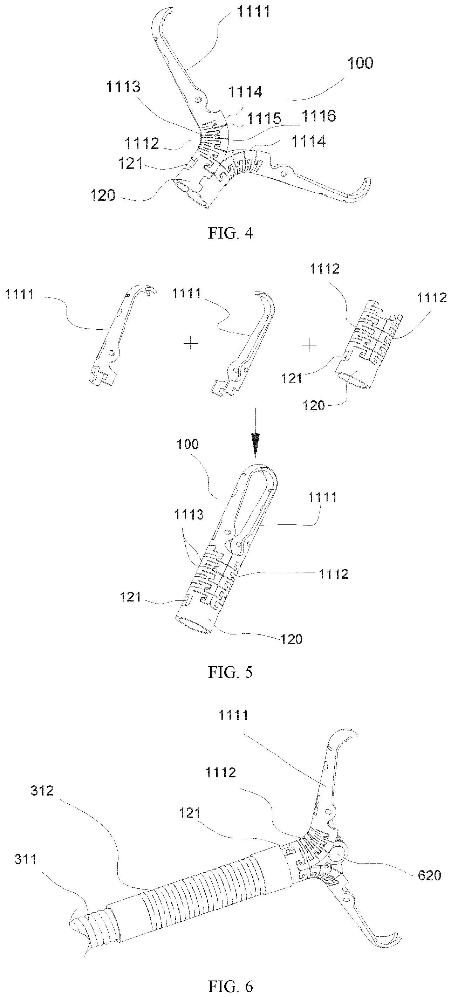

Referring to FIGS. 3 and 4, in an embodiment, the gripper 100 has an integral structure assembled from a plurality of parts. The gripping arms 110 are separate structures. That is, the gripping arms 110 are separate components, and then are connected to each other and assembled into the integral structure. The connection between the gripping arms 110 is generally a fixed connection. However, in some embodiments, a certain movement space may be provided between the gripping arms 110 to meet different functional requirements. In the same gripping arm 110, the gripping head 1111 and the bendable portion 1112 have an integrally formed structure. Certainly, in some embodiments, reference may alternatively be made to an assembly relationship between the gripping arms 110. The gripping head 1111 and the bendable portion 1112 in the same gripping arm 110 are separate structures, and are then connected to each other to form the integral gripping arm 110.

Referring to FIG. 5, in another embodiment, the gripping head 1111 and the bendable portion 1112 in each gripping arm 110 are separate structures. That is, the gripping head 1111 and the bendable portion 1112 are two separate parts and are then connected to each other to form the integral gripping arm 110. As shown in FIG. 5, the bendable portions 1112 of different gripping arms 110 are connected into an entirety as an integrally formed structure, which facilitates integral molding and machining and may also simplify assembling steps. Certainly, in other embodiments, as shown in FIG. 3, the bendable portions 1112 of different gripping arms 110 may be separated from each other and then assembled into an entirety.

In the gripper 100, through the above various embodiments, some parts are disassembled into separate structures, which are manufactured separately, this can reduce manufacturing difficulty and costs of individual parts. The above parts may be connected by welding, bonding, mechanical structural connection (such as engagement, screwing, or riveting), or the like.

Referring to FIGS. 1 to 5, in an embodiment, the bendable portion 1112 has a semi-cylindrical structure. When the gripper 100 closes, the bendable portions 1112 can enclose a cylindrical structure. The semi-cylindrical shape refers to a non-complete cylindrical shape, which may not necessarily be half of the cylindrical structure and may be one-third or other proportions of the entire cylindrical structure. In addition, in other embodiments, the bendable portion 1112 may alternatively have another structure, such as a sheet shape, which is not limited to the semi-cylindrical structure.

Further, the gripping arms 110 are arranged in a clamping jaw structure to grip a target. The bendable portion 1112 has a deformable structure capable of bending in a closing direction of the gripper 100 and/or bending in an opening direction of the gripper 100. The moving member 200 is movably arranged and is connected to the gripper 100. The gripper 100 opens and closes based on a change in a position of the moving member 200. In FIGS. 6 to 9 and FIGS. 12 to 15, the moving member 200 is a pull rod. In other embodiments, the moving member 200 may alternatively have another structure. The moving member 200 is connected to the gripper 100. Movement of the moving member 200 can control movement of the gripper 100 in the opening direction and the gripping direction.

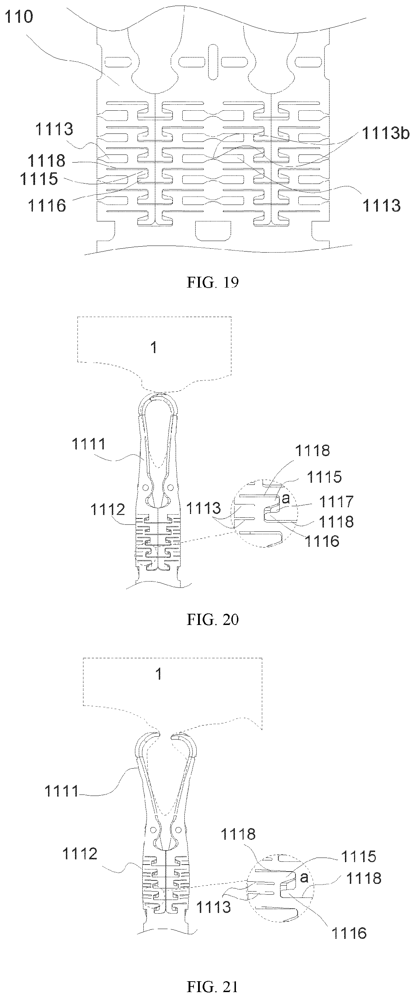

The clamping jaw structure is a structure that can firmly grip the target. For example, in FIGS. 1 to 13, when two gripping arms 110 are provided, the two gripping arms 110 face each other. When the two gripping arms 110 are closed (in a gripping state in this case) as shown in FIG. 1, the target can be gripped, as shown in FIGS. 20 and 21. In other embodiments, when different numbers of gripping arms 110 are provided, there may have different clamping jaw structures. For example, when three gripping arms 110 are provided, the three gripping arms 110 may be arranged in a triangle to grip the target.

Unlike the condition that the gripping arms 110 are limited by the sleeve to realize opening and closing of the gripping arms 110 in the prior art, in this embodiment, the opening and closing of the gripping arms 110 mainly rely on deformation of the bendable portion 1112. The bendable portion 1112 has a deformable structure capable of bending in the closing direction of the gripper 100 and/or bending in the opening direction of the gripper 100. Referring to FIG. 1, in the embodiment illustrated, an initial state of the gripper 100 is a gripping state. That is, when the bendable portion 1112 is not deformed, the gripper 100 is in the gripping state. In this case, the bendable portion 1112 has at least a deformable structure that can bend in the opening direction of the gripper 100, so as to realize the opening of the gripper 100 as shown in FIG. 2. Certainly, in other embodiments, the initial state of the gripper 100 may alternatively be an opened state. That is, when the bendable portion 1112 is not deformed, the gripper 100 is in an opened state shown in FIG. 2. In this case, the bendable portion 1112 has at least a deformable structure that can bend in the closing direction of the gripper 100, so as to move to the state as shown in FIG. 1 to realize the closing of the gripper 100.

The gripping head 1111 has higher resistance to bending deformation than the bendable portion 1112 to ensure that the gripping arms 111 provide a better holding effect on the target. Bending deformation of the bendable portion 1112 is achieved through structural deformation thereof, which may be achieved by, for example, arranging shrinkage slits capable of shrinkage and deformation on the bendable portion 1112, or by changing a thickness of the material of the bendable portion 1112, or by selecting a material easier to deform, and certainly, may alternatively be achieved by another structure. This will be introduced in more detail hereinafter. The bending deformation of the bendable portion 1112 is reversible. That is, the bendable portion 1112 has elasticity and can rebound and reset when the external force is removed, so the bending deformation can be repeated.

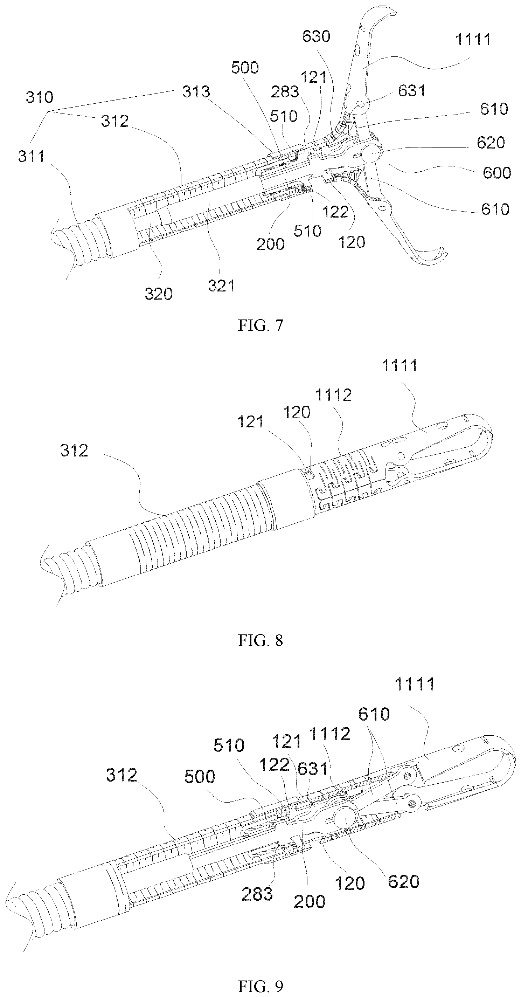

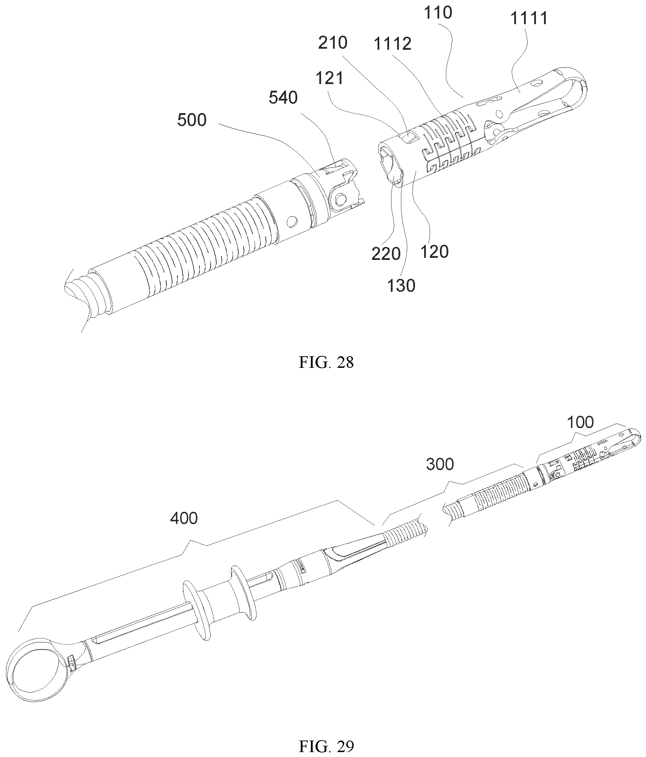

The movement of the moving member 200 may be a movement in an axial direction thereof, rotational movement, or the like. For example, referring to FIGS. 6, 7, FIGS. 12 and 13, in an embodiment, when the moving member 200 moves away from a control handle 400 (as shown in FIG. 29) in the axial direction thereof and approaching the gripper 100 (moves rightward as shown in the figure), the moving member 200 can drive the gripper 100 to open outwards, thereby causing the gripper 100 to move to the opened state. Referring to FIGS. 8, 9 and FIGS. 14 and 15, in an embodiment, when the moving member 200 moves approaching the control handle 400 in the axial direction thereof and away from the gripper 100 (moves leftward as shown in the figure), the moving member 200 can drive the gripper 100 to approach each other inwardly, thereby causing the gripper 100 to move to the gripping state. Certainly, in other embodiments, a movement relationship of the moving member 200 and a movement relationship of the gripper 100 may be different from those shown in FIGS. 6 to 9 and FIGS. 12 to 15. For example, when the moving member 200 moves towards the control handle 400, the gripper 100 is driven to open, and when the moving member moves towards the gripper 100, the gripper 100 is driven to close.

In the structures shown in the above embodiments, the sleeve in the existing structure is omitted, and the moving member 200 directly drives the gripper 100, so as to realize the opening and closing of the gripper 100 based on a deformable state of the bendable portion 1112. Since the sleeve has a limiting effect on the gripping arms 110 and is omitted, the gripping arm 110 starts to deform from the bendable portion 1112, and a deformation region of the gripping arm 110 is closer to the bottom of the entire gripper 100. Therefore, under the same open magnitude requirement, a length of the gripper 100 is shorter than that of a structure combining the gripping arms 110 and the sleeve in the prior art. Under the same length, the gripper 100 can open to a larger angle than the structure combining the gripping arms 110 and the sleeve in the prior art, making it easier to hold tissue of the target. In addition, the configuration of the gripper 100 prevents fit clearance of parts required for shaft hole fit or sliding displacement, so the gripping arms 110 have higher bending repetition accuracy.

Moreover, compared with the structure combining a plurality of parts in the existing hemostatic clamp (or tissue clamp), after the use of the gripper 100, the structure is simpler, the assembling requirement is lower, the cost is greatly reduced, and the control accuracy is higher. Similarly, the length of the entire gripper 100 is shorter than that of the existing hemostatic clamp (or tissue clamp). Since an inner diameter of an endoscopic instrument channel is very limited, it is easier for this shorter gripper 100 to extend through the endoscopic instrument channel.

Further, as described above, bending deformation of the bendable portion 1112 is achieved through an integrated structure thereof. Referring to FIGS. 1 to 5, in some embodiments, an end of the gripper 100 where the gripping head 1111 is located is a distal end, and an end away from the gripping head 1111 is a proximal end. A direction from the proximal end of the gripper 100 to the distal end thereof is a longitudinal direction of the gripper 100. In order to realize the deformable structure, the deformable structure includes a plurality of first shrinkage slits 1113. The first shrinkage slits 1113 are arranged in sequence in the longitudinal direction.

In an embodiment, as shown in FIG. 1, the gripper 100 in the initial state is maintained in the gripping state, the first shrinkage slits 1113 are maintained in the initial state, and each part of the bendable portion 1112 is not deformed. As shown in FIG. 2, when the gripper 100 is required to open, the bendable portion 1112 is deformed outwards, and the first shrinkage slit 1113 shrinks and deforms, thereby shrinking an outer side of the bendable portion 1112 (the sides of the gripping arms 110 away from each other), so that the entire gripping head 1111 opens.

Referring to FIGS. 1 to 5 and FIGS. 16 to 19, in an embodiment, the first shrinkage slit 1113 extends in a circumferential direction of the bendable portion 1112. The first shrinkage slits 1113 are arranged parallel to each other. Certainly, in addition to being parallel to each other, the first shrinkage slits 1113 may also be arranged in other non-parallel manners. The first shrinkage slits 1113 are all arranged in the circumferential direction of the bendable portion 1112 in parallel, which can unify bending deformation directions of the first shrinkage slits 1113, so that the bending deformation of the gripper 100 is smoother and more stable.

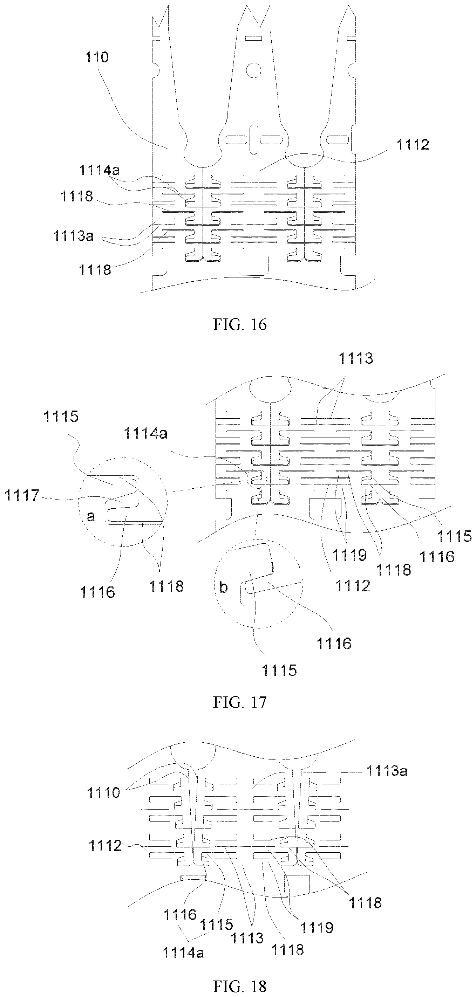

To achieve smoother bending changes, in an embodiment, the first shrinkage slits 1113 are divided into five groups, and each group of first shrinkage slits 1113a includes at least one first shrinkage slit 1113. Referring to FIGS. 1 to 5 and FIGS. 16 to 17, in this embodiment, each group of first shrinkage slits 1113a includes two first shrinkage slits 1113. As shown in FIGS. 18 and 19, in this embodiment, each group of first shrinkage slits 1113a includes one first shrinkage slit 1113. Shrinkage of each first shrinkage slit 1113 enables the bendable portion 1112 to have a certain bending angle. In combination with a plurality of groups of first shrinkage slits 1113, the bendable portion 1112 may have a larger opening/closing angle. A length formed by a combination of all the first shrinkage slits 1113 in the longitudinal direction determines a bending deformation region of the whole bendable portion 1112. The number of the groups of first shrinkage slits 1113a, the gap between two adjacent groups of first shrinkage slits 1113a in the longitudinal direction, and the number of the first shrinkage slits 1113 in the group of first shrinkage slits 1113a may be flexibly set as required. For example, 4 to 6 groups of first shrinkage slits 1113a may be provided.

Referring to FIG. 19, in an embodiment, the first shrinkage slit 1113 is in the shape of an elongated groove. A middle part of the first shrinkage slit 1113 has two arc-shaped edges 1113b protruding relative to each other. When the gripper 100 is opened to a limited position, the arc-shaped edges 1113b may contact each other, thereby determining a maximum opening angle. When the gripper 100 is in the gripping state, the arc-shaped edges 1113b may contact each other, thereby providing a support force for the gripper 100.

In consideration of a requirement for minimally invasive surgery, the gripping instrument generally has a very delicate and compact structure. Therefore, on the premise of meeting a small volume of the gripping instrument, it is generally inappropriate that the gripper 100 is made of a material with a larger thickness. However, a thinner thickness requirement may result in weakening of strength of the bendable portion 1112. Specifically, as shown in FIGS. 2 and 4, when the external force exerted by an operator is excessively large, the gripping arm 110 bends outwards at an excessive angle, and the gripping arm 110 may be broken at the bendable portion 1112. Based on this, in an embodiment, as shown in FIGS. 2 and 4, the bendable portion 1112 has a limiting structure 1114. The limiting structure 1114 is configured to limit a maximum angle at which the bendable portion 1112 is bent in the opening direction. That is, within the maximum angle, the bendable portion 1112 may be bent freely. When the bending angle thereof reaches the maximum angle, the limiting structure 1114 begins to function, restricting the bendable portion 1112 from continuing to bend outwards, thereby protecting the bendable portion 1112 and the gripper 100. The limiting structure 1114 limits the maximum angle mainly by position limiting in the longitudinal direction of the gripper 100.

Referring to FIGS. 2 and 4, and FIGS. 16 to 19, in an embodiment, each limiting structure 1114 includes a plurality of limiting elements 1114a arranged in the longitudinal direction of the gripper 100. The limiting element 1114a includes a first limiting block 1115 and a second limiting block 1116 facing each other. As shown in two partial enlarged views of FIG. 17a and 17b, gaps 1117 arranged along the longitudinal direction are provided between the first limiting blocks 1115 and the second limiting blocks 1116 respectively. During the bending of the bendable portion 1112 in the opening direction, the first limiting block 1115 and the second limiting block 1116 approach each other and form a buckling structure (see the partial enlarged view shown in FIG. 17b). That is, in the initial state, the gap 1117 is left between the first limiting block 1115 and the second limiting block 1116 as shown in FIG. 17a. When the gripper 100 gradually opens outwards, the first limiting block 1115 and the second limiting block 1116 move relative to each other in the longitudinal direction, and the gap 1117 gradually becomes smaller. Finally, when the bendable portion 1112 reaches the maximum angle, as shown in FIG. 17b, the first limiting block 1115 and the second limiting block 1116 fit with each other to form position limiting.

As shown in FIGS. 16 to 19, in an embodiment, the first limiting block 1115 and the second limiting block 1116 are two limiting hook structures that interlock with each other. The limiting hook structure may alternatively be replaced with other structures with similar functions. Referring to FIGS. 2 and 4, in an embodiment, the first shrinkage slit 1113 is located in the middle of the bendable portion 1112 in the circumferential direction thereof. At least two groups of limiting structures 1114 are provided. In the circumferential direction of the bendable portion 1112, the bendable portion 1112 is provided with the limiting structures 1114 on two sides respectively, which can further ensure that the entire bendable portion 1112 can synchronize bending changes and position limiting.

Referring to FIGS. 2 and 4 and FIGS. 16 to 19, in an embodiment, in the same limiting element 1114a, the first limiting blocks 1115 and the second limiting blocks 1116 are formed by cutting in the sidewalls of the bendable portion 1112 located on sides of the first shrinkage slit 1113. First ends of the first limiting block 1115 and the second limiting block 1116 adjacent to the first shrinkage slit 1113 are connected into an entirety, and second ends of the first limiting block 1115 and the second limiting block 1116 are separated from each other. When the gripper 100 opens outwards, the first limiting block 1115 and the second limiting block 1116 may also open along with the gripper 100.

As shown in FIGS. 2 and 4 and FIGS. 16 to 19, each group of first shrinkage slits 1113a may be aligned with one limiting element 1114a in the circumferential direction, thereby ensuring that a limiting effect of the limiting element 1114a can accurately act on the corresponding first shrinkage slit 1113, to prevent breakage of the bendable portion 1112 caused by continuous shrinkage and deformation of the first shrinkage slit 1113 after the bendable portion bends to the maximum angle.

The number of the limiting elements 1114a may be greater than that of groups of the first shrinkage slits 1113a, thereby completely covering all the first shrinkage slits 1113 in the longitudinal direction, to achieve a better limiting effect. Certainly, the number of the limiting elements 1114a may alternatively be less than or equal to that of the groups of first shrinkage slits 1113a.

Further, referring to FIGS. 16 to 18, in an embodiment, second shrinkage slits 1118 at least partially arranged along the circumferential direction of the bendable portion 1112 are provided between the first limiting block 1115 and the second limiting block 1116. The second shrinkage slit 1118 can separate the first limiting block 1115 from the second limiting block 1116 so that the first limiting block 1115 and the second limiting block 1116 can move relative to each other. The second shrinkage slit 1118 is in communication with the gap 1117 between the first limiting block 1115 and the second limiting block 1116.

Bending motion of the gripper 100 in the opening direction and bending motion of the gripper 100 in the gripping direction are often accompanied by twist motion around the circumferential direction thereof. Therefore, referring to FIGS. 16 to 19, in an embodiment, two ends of each group of first shrinkage slits 1113a respectively extend between two adjacent second shrinkage slits 1118 of the limiting element 1114a in the longitudinal direction. An overlapping region between the first shrinkage slit 1113 and the second shrinkage slit 1118 forms a twist deformation section 1119, so that the bendable portion 1112 is capable of bending and twist deformation. When the twist deformation section 1119 is provided, the bending deformation of the gripper 100 is smoother, and breakage of the bendable portion 1112 due to a twisting force is also prevented. By adjusting a circumferential length and a longitudinal height of the twist deformation section 1119, a maximum opening angle, bending softness or support of the bendable portion 1112 can be further changed, which may be flexibly set according to actual requirements. For example, the twist deformation section 1119 may also play a role in limiting the maximum opening angle through an own structure. This limiting effect may also be combined with the above limiting structure 1114, to jointly define a maximum opening/closing angle of the bendable portion 1112.

In the embodiments shown in FIG. 17 and FIG. 19, the second shrinkage slit 1118 is shaped as a straight line. In the embodiments shown in FIG. 18, the second shrinkage slit 1118 is U shaped.

Further, as described above, after the gripper 100 grips a target 1, the moving member 200 is required to move together with the gripper 100 to a predetermined locking structure for locking. However, in actual use, when the gripper 100 grips human tissue with different hardness or thicknesses (as shown in FIGS. 20 and 21), the closing angle of the gripper 100 may be limited. Since the closing angle is related to a stroke of the moving member 200, the gripper 100 and the moving member 200 cannot move to the position of the locking structure, and the gripper 100 cannot be maintained in the gripping state.

With respect to the problem, referring to FIGS. 20 and 21, the second shrinkage slit 1118 is provided with a gap in the longitudinal direction. The second shrinkage slits 1118 in the limiting structures 1114 form an adaptive floating structure that can be deformed in the gripping direction, so that, when the gripper 100 grips the target 1, the bendable portion 1112 can be adaptively bent and deformed in the closing direction according to a volume of the target 1, providing an adaptive stroke range for the moving member 200 and the gripper 100, increasing a rigid deformation margin of the moving member 200, and always enabling the moving member 200 and the gripper 100 to move to a lock position of the locking structure, thereby achieving accurate and reliable locking.

Specifically, referring to FIG. 20, when the gripper 100 grips a thinner target 1, the gripper 100 may close normally, and the second shrinkage slit 1118 maintains a normal gap (as shown in the partial enlarged view of FIG. 20a). The moving member 200 and the gripper 100 may accurately move to the position of the locking structure as shown in the above locking stroke, and lock the gripper 100 in the gripping state. Referring to FIG. 21, when the gripper 100 grips a thicker target 1, the gripper 100 cannot be closed to the extent shown in FIG. 20. In this case, the moving member 200 may be continuously pulled, and the second shrinkage slit 1118 can be deformed in the closing direction of the gripper 100 (as shown in the partial enlarged view of FIG. 21a). For example, in an embodiment, each second shrinkage slit 1118 can provide an amount of compression in the range of 0.02 to 0.05 MM in the longitudinal direction. With the number of the second shrinkage slits 1118 as shown in the figure, an amount of deformation of nearly 0.1 to 0.2 mm may be provided after accumulation of a plurality of second shrinkage slits 1118, thereby causing the bendable portion 1112 to bend inwards as shown in FIG. 21 (in FIG. 21, two sides of the bendable portion 1112 are slightly deformed and bulge outwards), and compensating for a lost stroke of the gripper 100, so that the gripper 100 can finally be locked to the locking structure.

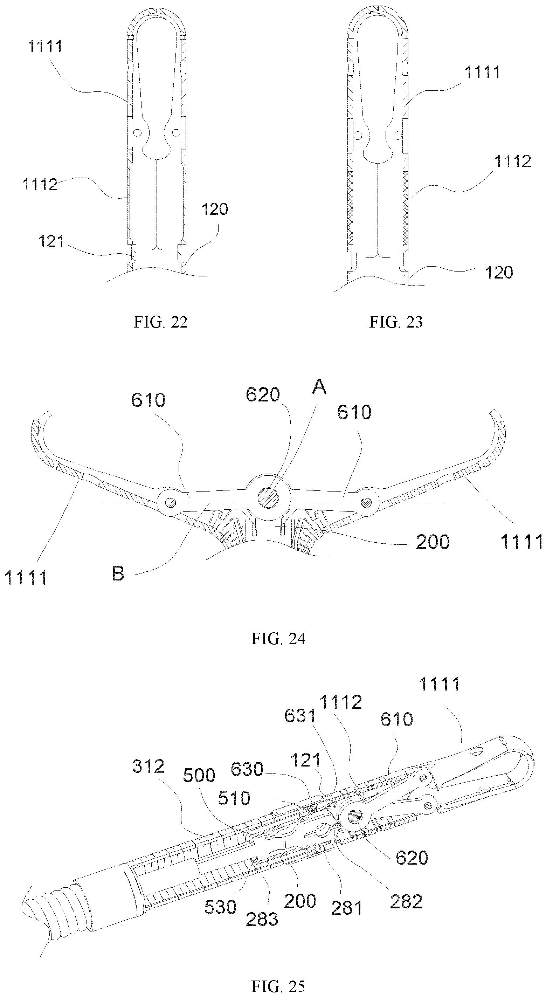

In the above embodiments, a structure in which the bendable portion 1112 is provided with shrinkage slits to achieve bending deformation. In this embodiment, the deformable structure of the bendable portion 1112 is not limited thereto, which may be implemented in other manners. For example, referring to FIG. 22, in an embodiment, in the gripper 100, the bendable portion 1112 may alternatively be thinner than other portions, which is, for example, thinner than the gripping head 1111 and a connecting portion 120, so that, when a moving rod 200 drives the gripper 100 to move, the bendable portion 1112 can bend and deform preferentially.

Referring to FIG. 23, in an embodiment, in the gripper 100, the bendable portion 1112 may have a material and a structure that are easier to bend and deform, for example, a material that is easier to bend and deform than the gripping head 1111 and the connecting portion 120 (which will be described in detail later), such as a metal material, a plastic material, or woven wire cloth with better bending performance, so that the bendable portion 1112 can be bent and deformed preferentially when the moving rod 200 drives the gripper 100 to move. Certainly, the bendable portion 1112 may alternatively adopt another structure with better bending deformation performance, such as a metal braided structure.

Further, in order to reduce mutual interference between the grippers 100 during the closing, in an embodiment, opposite ends of the grippers 100 are provided with avoidance structures 1110 so that the opposite ends can avoid each other when the grippers 100 are closed. Referring to FIG. 18, in this embodiment, the opposite ends of the gripper 100 retract inwards to form the avoidance structure 1110. The avoidance groove is formed between two opposite grippers 100 through the retracting region. A width of the avoidance groove gradually increases in the longitudinal direction of the gripper 100. An end of the avoidance groove adjacent to the gripping head 1111 is wider than another end of the avoidance groove. Certainly, the avoidance structure 1110 may alternatively be other structures that can play an avoidance function, and which is not limited to the structure shown in the figures.

Further, the moving member 200 may be connected to the gripper 100 through various forms of structures, as along as the structures can drive the gripper 100 to move in the opening direction and the gripping direction. The moving member 200 may be directly connected to the gripper 100, or may be connected to the gripper 100 through a gripper connection structure 600.

Referring to FIGS. 7, 13, and 24, in an embodiment, the gripper connection structure 600 includes two connecting rods 610. Each of the two connecting rods 610 has an end connected to the distal end of the moving member 200 and rotatable around a shaft 620 and another end connected to a cross shaft on the gripping head 1111 and also rotatable around the cross shaft. The connecting rods 610 assembly is roughly Y-shaped, in order for effectively transferring push and pull forces of the moving member 200 generated by up-and-down motion of the moving member 200 to the gripping head 1111, so as to realize control over the opening and closing of the gripping head 1111.

Referring to FIG. 21, as the moving member 200 moves towards an end away from the control handle 400, the gripper 100 can be opened in the opening direction. A rotation center A of the connecting rod 610 and the moving member 200 can cross over a connecting line B between two rotation centers between the connecting rods 610 and the gripping heads 1111, thereby forming self-locking, so that the gripper 100 is kept in an opened state and cannot be easily closed by an external force, and the gripping head 1111 can only be controlled to retract through the control handle 400.

Certainly, the gripper connection structure 600 may alternatively be connected by using another structure, for example, in various connection manners between the gripping arm 110 and the pull rod disclosed in the prior art.

Further, referring to FIG. 29, in this embodiment, a process in which the gripper 100 is separated from and temporarily stays in the body of the subject is briefly described based on an example in which the insertable gripping instrument is a clamp device.

Referring to FIGS. 6 to 9, FIGS. 12 to 15, and FIG. 29, in addition to the gripper 100 and the moving member 200, the insertable gripping instrument further includes a transmission assembly 300, a control handle 400, and a separation base 500.

The transmission assembly 300 plays roles in supporting the gripper 100 and transferring motion and force to the moving member 200. Referring to FIG. 7, the transmission assembly 300 includes a sleeve assembly 310 and a transmission member 320 extending in the sleeve assembly 310. The transmission member 320 is connected to the moving member 200. The gripper 100 and the separation base 500 are detachably connected or integrally formed, to ensure that the gripper 100 can be separated from the separation base 500 when needed.

The separation base 500 is rotatably connected to the sleeve assembly 310. For example, as shown in FIG. 7, the separation base 500 is sleeved in the sleeve assembly 310. The connection between the separation base 500 and the gripper 100 allows the separation base 500 and the gripper 100 to rotate in a circumferential direction of the sleeve assembly 310, so that the gripper 100 can rotate relative to the sleeve assembly 310 as a whole. The sleeve assembly 310 is connected to the control handle 400. The control handle 400 and the transmission member 320 form a linkage structure to control actions of the transmission member 320, the moving member 200, and the gripper 100. For example, the operator may control rotation of the gripper 100 relative to the sleeve assembly 310 through the control handle 400, and may also control the opening and closing of the gripper 100 through the control handle 400.

No matter how the moving member 200 moves, the moving member 200 has a first stroke, a second stroke, and a third stroke. The first stroke, the second stroke, and the third stroke are three parts of the entire motion stroke of the moving member 200. The three strokes may be in a same direction, or at least two strokes may be in different directions.

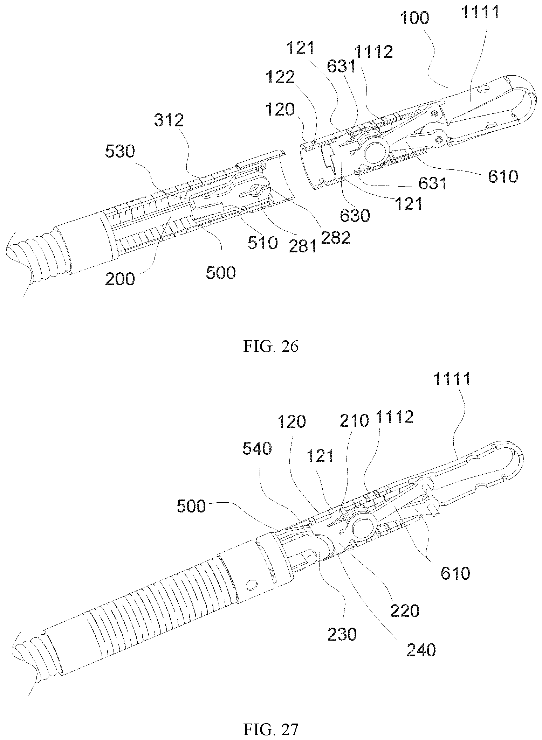

Referring to FIGS. 6 to 9 and FIGS. 25 and 26, in an embodiment, the moving member 200 achieves internal disengagement through a combination of split structures. Specifically, the moving member 200 and the gripper 100 have a snap-fit connection structure. Referring to FIGS. 25 and 26, the moving member 200 has an engaging groove 281. The engaging groove 281 has an opening 282 having a size smaller than a groove cavity thereof. The moving member 200 is engaged with the gripper connection structure 600 through the engaging groove 281, and may specifically be connected to the mounting shaft 620 of the connecting rod 610.

When the moving member 200 moves along the first stroke and the second stroke, limited by a blocking effect of the opening 282, the gripper connection structure 600 and the gripper 100 may move together with the moving member 200 to realize the opening and gripping of the gripper 100.

Referring to FIGS. 6 and 7, in this case, the moving member 200 is in the first stroke, and when the moving member 200 moves away from the control handle 400 in the axial direction thereof and approaching the gripper 100 (rightward in the figure), the moving member 200 can drive the gripper 100 to open outwards, thereby causing the gripper 100 to move to the opened state.

Referring to FIGS. 8 and 9, in this case, the moving member 200 is in the second stroke, and when the moving member 200 moves approaching the control handle 400 in the axial direction thereof and away from the gripper 100 (leftward in the figure), the moving member 200 can drive the gripper 100 to approach each other inwardly, thereby causing the gripper 100 to move to the gripping state.

As shown in FIGS. 25 and 26, after the moving member 200 is in the third stroke, the gripper 100 and the gripper connection structure 600 are blocked and cannot continuously move towards the control handle 400. In this case, under an external force, the gripper connection structure 600 is separated from the opening 282 of the engaging groove 281, and then the moving member 200 is separated from the gripper connection structure 600 accordingly, thereby achieving inner disengagement. The moving member 200 may not continue to drive the gripper 100 to move, so that without the control over the gripper 100, and the gripper 100 is kept in a locked state.

Referring to FIG. 26, when the moving member 200 moves a position shown in the figure, the gripper 100 and the separation base 500 are separated from each other. At this point, the gripper 100 is left on the target that is gripped. The separation base 500, the moving member 200, and the transmission assembly 300 may be withdrawn from the body of the subject. In this process, the motion stroke of the moving member 200 is an outer disengagement stroke.

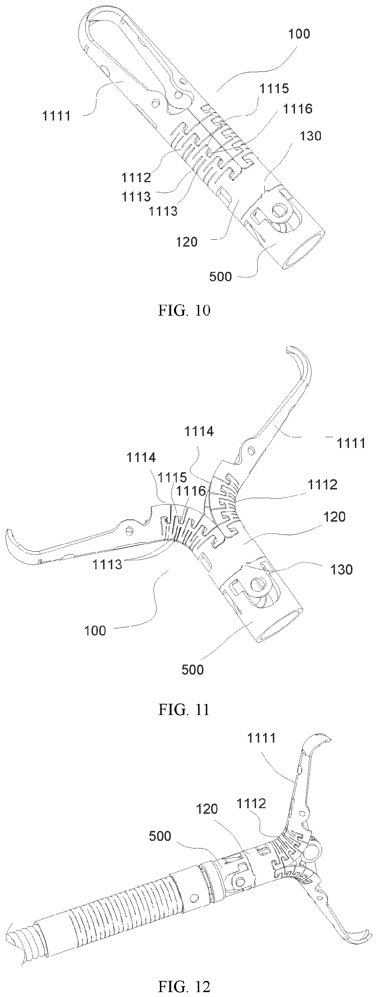

In another inner disengagement structure, referring to FIGS. 12 to 15 and FIGS. 27 and 28, the moving rod 200 has an integrally formed structure, which has a retaining section 220 and a separation section 230. The retaining section 220 and the separation section 230 are connected into an entirety through a second tearing portion 240.

The retaining section 220 is connected to a gripping body 110 through the gripper connection structure 600. A locking fitting portion 210 is located on the retaining section 220 or on the gripper connection structure 600. As shown in FIG. 27, when the moving rod 200 is in the third stroke, the second tearing portion 240 is broken, and the retaining section 220 is separated from the separation section 230. The retaining section 220 and the gripping body 110 are left in the body of the subject, and the separation section 230 is removed from the body of the subject together with the separation base 500.

In this embodiment, the retaining section 220 and the separation section 230 are integrally formed. That is, the entire moving rod 200 is integrally machined with the same material and is not formed by assembling two or more parts. Even, the locking fitting portion 210, the retaining section 220 and the separation section 230 may also be integrally formed. The moving rod 200 is easy to manufacture, does not require secondary assembly, and has a lower cost and better stability. A width of the second tearing portion 240 may be reserved as required. A plurality of second tearing portions 240 may also be provided according to functional requirements, which can make the structure more reliable and stable.

In order to assist the moving rod 200 in inner disengagement, referring to FIGS. 27 and 28, in an embodiment, the separation base 500 has a stop structure 540. The stop structure 540 is located on a movement path of the retaining section 220. When the moving rod 200 moves along the third stroke, the stop structure 540 prevents the retaining section 220 from continuously moving with the separation section 230, to help separation of the retaining section 220 from the separation section 230.

In the embodiment shown in FIG. 24, the moving rod200 has a slot arranged in the axial direction thereof. The stop structure 540 protrudes towards the moving rod 200 and extends into the slot to abut against a wall of the slot when the moving rod 200 moves along the inner disengagement stroke.

Further, referring to FIGS. 1 to 5 and FIGS. 10 to 11, in an embodiment, the gripper 100 includes a connecting portion 120. The connecting portion 120, the bendable portion 1112, and the gripping head 1111 are sequentially connected into an entirety. As shown in FIGS. 6 to 9, the separation base 500 is removably connected to the connecting portion 120, or as shown in FIGS. 10 to 11, the separation base 500 and the connecting portion 120 are integrally formed.

Referring to FIGS. 25 to 28, the connecting portion 120 has the locking structure 121 as described above. The locking structure 121 is configured to lock the gripper 100 in the gripping state. Certainly, in other embodiments, the gripper 100 may not include the connecting portion 120, and the locking structure 121 may be directly arranged on the bendable portion 1112 or other structures. The locking structure 121 is configured to lock the gripper 100 in the gripping state. The locking structure 121 can at least prevent the gripper 100 from moving in the opening direction to ensure that the gripper 100 is always in the gripping state.

In an embodiment, the moving member 200 or the gripper connection structure 600 has a locking fitting portion 631 or 210. When the moving member 200 moves along the third stroke, the locking structure 121 is located on a moving path of the locking fitting portion 631 or 210. When the locking fitting portion 631 or 210 moves to the locking structure 121, the locking fitting portion 631 or 210 and the locking structure 121 are in locking fit to keep the gripper 100 in the gripping state.

Referring to FIGS. 25 to 28, in an embodiment, the gripper 100 forms a cylindrical structure. An end of the moving member 200 extends into the cylindrical structure and is connected to the gripper 100. The locking fitting portion 631 or 210 includes an elastic piece protruding towards the gripper 100. The locking structure 121 includes an engaging slot that can fit with the elastic piece. The elastic piece is inclined towards a distal side of the gripper 100 in a protruding direction thereof. The inclined elastic piece can move towards the control handle 400 along an inner wall of the gripper 100 to prevent the elastic piece from being engaged in other parts of the gripper 100. When the elastic piece moves to the position corresponding to the engaging slot, the elastic piece may be engaged into the engaging slot under an elastic force, to prevent the gripping state from being opened due to retraction of the moving member 200 and the gripper 100.

Specifically, referring to FIG. 25, in an embodiment, the locking fitting portion 631 is arranged on a cylindrical body 630. The cylindrical body 630 is part of the gripper connection structure 600, and may form a linkage structure with the connecting rod 610 through the shaft 620. The moving member 200 extends through the cylindrical body 630 and is connected to the shaft 620. The cylindrical body 630 can follow the moving member 200 and move towards the control handle 400 until it is locked by the locking structure.

Certainly, the elastic piece shown in the figure is just an example of the locking fitting portion 631 or 210. In other embodiments, other structures that may achieve a locking function may alternatively be adopted. For example, a manner in which the gripping arm 110 or the push rod and the sleeve are locked disclosed in the prior art may be adopted.

In order to more stably lock the gripper 100, referring to FIGS. 25 to 28, in an embodiment, more than two locking structures 121 and more than two locking fitting portions 631 or 210 are provided, and two locking structures 121 and two locking fitting portions 631 or 210 are shown in the figure. In order to receive uniform force, in an embodiment, the locking structures 121 are evenly distributed on the gripper 100 in the circumferential direction of the gripper 100 (that is, adjacent locking structures 121 are spaced apart at the same angle). The locking fitting portions 631 or 210 face the gripper 100, which may alternatively, for example, evenly distributed on the moving member 200 in the circumferential direction of the moving member 200.

Further, referring to FIGS. 25 and 26, in an embodiment, in order to mount the separation base 500, the connecting portion 120 is provided with an engaging portion 122, and the separation base 500 has a leg 510. The leg 510 is engaged with the engaging portion 122. The moving member 200 has a pushing portion 283. When the moving member 200 moves along the third stroke, the separation base 500 is arranged on a moving path of the pushing portion 283. The pushing portion 283 can push the separation base 500 to move towards the control handle 400 to separate the separation base 500 120 from the connecting portion. Referring to FIGS. 25 and 26, for example, the separation base 500 has a protruding portion 530. The protruding portion 530 is located on the moving path of the pushing portion 283. The protruding portion 530 may be the bottom of the cylindrical separation base 500, and the bottom has a through hole through which the moving member 200 extends through. In order to engage the separation base 500 with the connecting portion 120, the leg 510 thereof may be provided with a bent buckle. The engaging portion 122 may be a slot fitting with the buckle. The buckle extends into the slot for achieving a snap-fit connection. Pushed by the pushing portion 283, the buckle may be deformed, to be disengaged from the engaging portion 122.

Further, referring to FIG. 7, in terms of the transmission assembly 300, the sleeve assembly 310 may generally include a spring support sleeve 311. The transmission member 320 (e.g., a traction control wire) extends in the spring support sleeve 311. The moving member 200 may be fixedly connected to the transmission member 320 through a reduction sleeve 321 or other structures. A connecting tube 312 fits over the spring support sleeve 311, and is fixed to the spring support sleeve 311. The connecting tube 312 is provided with a fixed base 313. The connecting tube 312 is rotationally connected to the separation base 500 and the gripper 100, so that the entire gripper 100 can rotate relative to the transmission assembly 300 along with the separation base 500.

The present application is illustrated above by using specific examples, which are only used to help understand the present application and are not intended to limit the present application. Those skilled in the art can also make various simple deductions, variants or substitutions based on the idea of the present application.

## Claims

1. An insertable gripping instrument, comprising:
a gripper comprising at least two gripping arms, the gripping arms being connected to each other, each gripping arm comprising a gripping head and a bendable portion, the gripping head and the bendable portion being connected to each other, and the gripping arm being configured to grip a target; the bendable portion having a deformable structure capable of bending in a closing direction of the gripper and/or bending in an opening direction of the gripper; and
a moving member movably arranged and being connected to the gripper,
wherein the gripper opens and closes based on a change in a position of the moving member.

2. The insertable gripping instrument according to claim 1, wherein the deformable structure comprises a plurality of first shrinkage slits arranged sequentially in a longitudinal direction of the gripper.

3. The insertable gripping instrument according to claim 2, wherein the first shrinkage slits are divided into a plurality of groups, each group of first shrinkage slits having at least one first shrinkage slit;
the bendable portion has a plurality of second shrinkage slits extending in a circumferential direction of the bendable portion, the second shrinkage slits being arranged in the longitudinal direction;
wherein two ends of each group of first shrinkage slits respectively extend between two adjacent second shrinkage slits in the longitudinal direction, and
an overlapping region between the first shrinkage slits and the second shrinkage slits form a twist deformation section, to cause the bendable portion to be capable of bending and twist deformation.

4. The insertable gripping instrument according to claim 3, wherein the first shrinkage slits extend in a circumferential direction of the bendable portion; and the bendable portion has a limiting structure configured to limit a maximum angle at which the bendable portion is bent in the opening direction.

5. The insertable gripping instrument according to claim 4, wherein each limiting structure comprises a plurality of limiting elements arranged in the longitudinal direction of the gripper;
wherein the limiting elements comprise first limiting blocks and second limiting blocks that face each other,
wherein gaps arranged in the longitudinal direction are provided between the first limiting blocks and the second limiting blocks, and the second shrinkage slits are in communication with the gaps; and
during a bending of the bendable portion in the opening direction, the first limiting blocks and the second limiting blocks approach each other and form a buckling structure.

6. The insertable gripping instrument according to claim 5, wherein in a same limiting element, each first limiting block and each second limiting block are formed by cutting in sidewalls of the bendable portion located on sides of a respective one of the first shrinkage slits, through the respective second shrinkage slits,
first ends of the first limiting block and the second limiting block adjacent to the first shrinkage slit are connected into an entirety, and second ends of the first limiting block and the second limiting block are separated from each other.

7. The insertable gripping instrument according to claim 6, wherein an initial state of the gripper is a gripping state; each second shrinkage slit has a gap in the longitudinal direction; and
the second shrinkage slits form an adaptive floating structure capable of deforming in a gripping direction, so that, when the gripper grips the target, the bendable portion is capable of being adaptively bent and deformed in the closing direction according to a volume of the target.

8. The insertable gripping instrument according to any one of claims 1 to 7, wherein opposite ends of the gripper are provided with avoidance structures to avoid each other when the gripper is closed.

9. The insertable gripping instrument according to claim 1, wherein the bendable portion is thinner than other parts of the gripper, so that the bendable portion is easier to be bent and deformed than the other parts.

10. The insertable gripping instrument according to any one of claims 1 to 9, wherein in the entire gripper, at least the gripping head and the bendable portion are integrally formed.

11. The insertable gripping instrument according to claim 1, wherein the bendable portion has a material and/or a structure that are/is easier to be bent and deformed than other parts of the gripper.

12. A gripper for an insertable gripping instrument, comprising at least two gripping arms that are connected to each other,
wherein each of the gripping arms comprises a gripping head and a bendable portion that are connected to each other, and
wherein the gripping arm is configured to grip a target; the bendable portion has a deformable structure capable of bending in a closing direction of the gripper and/or bending in an opening direction of the gripper.

13. The gripper according to claim 12, wherein the deformable structure comprises a plurality of first shrinkage slits arranged sequentially in a longitudinal direction of the gripper.

14. The gripper according to claim 13, wherein the bendable portion has a plurality of second shrinkage slits extending in a circumferential direction of the bendable portion, the second shrinkage slits being arranged in the longitudinal direction;
wherein two ends of each group of first shrinkage slits respectively extend between two adjacent second shrinkage slits in the longitudinal direction, and
an overlapping region between the first shrinkage slits and the second shrinkage slits form a twist deformation section, to cause the bendable portion to be capable of bending and twist deformation.

15. The gripper according to claim 14, wherein the first shrinkage slits extend in a circumferential direction of the bendable portion; the bendable portion has limiting structure; and
in the circumferential direction of the bendable portion, at least one side of the bendable portion is provided with the limiting structure.

16. The gripper according to claim 15, wherein each limiting structure comprises a plurality of limiting elements arranged in the longitudinal direction of the gripper,
wherein the limiting elements comprise first limiting blocks and second limiting blocks that face each other,
wherein gaps arranged in the longitudinal direction are provided between the first limiting blocks and the second limiting blocks, and the second shrinkage slits are in communication with the gaps; and
during a bending of the bendable portion in the opening direction, the first limiting blocks and the second limiting blocks approach each other and form a buckling structure.

17. The gripper according to claim 16, wherein in a same limiting element, each first limiting block and each second limiting block are formed by cutting in sidewalls of the bendable portion located on sides of a respective one of the first shrinkage slits, through the respective second shrinkage slits, and
first ends of the first limiting block and the second limiting block adjacent to the first shrinkage slit are connected into an entirety, and second ends of the first limiting block and the second limiting block are separated from each other.

18. The gripper according to claim 17, wherein an initial state of the gripper is a gripping state; each second shrinkage slit has a gap in the longitudinal direction; and
the second shrinkage slits in the limiting structures form an adaptive floating structure capable of deforming in a gripping direction, so that, when the gripper grips the target, the bendable portion is capable of being adaptively bent and deformed in the closing direction according to a volume of the target.

19. The gripper according to claim 12, wherein the bendable portion is thinner than other parts of the gripper, so that the bendable portion is easier to be bent and deformed than the other parts.

20. The gripper according to claim 12, wherein the bendable portion has a material and/or a structure that are/is easier to be bent and deformed than other parts of the gripper.
